# EUROPEAN PATENT SPECIFICATION

(11) **EP 4 545 103 B1**
(45) Date of publication and mention of the grant of the patent: **14.10.2026**
(21) Application number: 24209243.5
(22) Date of filing: 28.10.2024
(51) Int. Cl.: A61L 9/22, B64D 11/04, B60H 3/00, F24F 8/30

(54) **AIRCRAFT GALLEY WITH INTEGRATED AIR IONIZER**
FLUGZEUGBORDKÜCHE MIT INTEGRIERTEM LUFTIONISATOR
CUISINE DE BORD D'AÉRONEF AVEC IONISEUR D'AIR INTÉGRÉ

(30) Priority: 27.10.2023 IN 202341073227; 01.03.2024 US 202418593659
(43) Date of publication of application: 30.04.2025
(73) Proprietor: B/E Aerospace, Inc., Winston-Salem, NC 27105 (US)
(72) Inventor: GAJENDRA, Hemanth Raghav, Bangalore (IN); VASISHTA, Sujan Jayasimha, Bangalore (IN); KUPPAN, Skandan Berikai, Bangalore (IN)
(74) Representative: Dehns

(56) References cited:
- EP-A1- 3 925 884
- JP-A- 2004 012 087
- JP-A- 2011 168 159
- US-B2- 6 668 563

## Description

### FIELD CROSS-REFERENCE TO RELATED APPLICATIONS

The This application claims priority to, and the benefit of, India Provisional Patent Application No. 202341073227, filed October 27, 2023 and titled "AIRCRAFT GALLEY WITH INTEGRATED AIR IONIZER," and of US 18/593,659.

### FIELD

The present disclosure generally relates to systems for managing air quality in an aircraft, and more specifically, to using air ionizers in an aircraft.

### BACKGROUND

Travel within aircraft includes the recirculation of air within the aircraft. Circulation of air within an enclosed space, such as aircraft, may include circulation of harmful pathogens (e.g., viruses, bacteria, etc.) and undesirable odors. Currently, commercial aircraft use air filters, including high efficiency particulate air (HEPA) filters, to filter the air during recirculation. Generally, air filters are placed at central locations and therefore may not have continuous effectiveness within the enclosed space. Furthermore, more effective air filters, such as HEPA filters, come with an increased cost both in terms of the filter itself as well as the energy used to force air through the air filter. Furthermore, air filters, including HEPA filters, tend to not remove odors from the air within the enclosed space. JP 2004 021087 A discloses an air ionizer as part of an air conditioning unit. EP 3 925 884 A1 discloses a system for dispensing disinfectants in an air circulation system.

### SUMMARY

An aircraft galley is disclosed herein. The aircraft galley includes an air duct including an air inlet and an air outlet. The air outlet extends into the aircraft galley. The aircraft galley includes an ion emitter coupled to the air outlet. The aircraft galley includes a control unit electronically coupled to the ion emitter. The control unit is configured to control an ion output of the ion emitter. The air outlet includes an opening having an outer circumference and the ion emitter is coupled to the opening and extends inward from the outer circumference.

In various embodiments, the ion emitter is configured to be in an air flow passing through the air outlet. In various embodiments, the ion emitter is perpendicular to the air flow passing through the air outlet. In various embodiments, the aircraft galley further includes a plurality of ion emitters arranged around the outer circumference of the opening. In various embodiments, each of the plurality of ion emitters is perpendicular to a flow of air passing through the opening.

In various embodiments, the air inlet extends to an exterior of the aircraft galley to provide the air duct with air from outside of the aircraft galley. In various embodiments, the control unit includes a voltage input having a first voltage and a voltage output having a second voltage that is greater than the first voltage, the voltage output being coupled to the ion emitter.

Also disclosed herein is an aircraft. The aircraft includes a galley and an air duct extending into the galley. The air duct including an air outlet disposed within the galley. The aircraft includes an ion emitter coupled to the air outlet. The aircraft includes a control unit electronically coupled to the ion emitter, the control unit configured to control an ion generation of the ion emitter.

In various embodiments, the ion emitter is configured to be in an air flow passing through the air outlet and into the galley. In various embodiments, the ion emitter is perpendicular to the air flow passing through the air outlet. In various embodiments, the air outlet includes an opening having an outer circumference and the ion emitter is coupled to the opening and extends inward from the outer circumference. In various embodiments, the aircraft further includes a plurality of ion emitters arranged around the outer circumference of the opening. In various embodiments, each of the plurality of ion emitters is perpendicular to a flow of air passing through the opening.

In various embodiments, the galley further includes a wall disposed between the control unit and the galley. In various embodiments, the control unit includes a voltage input having a first voltage and a voltage output having a second voltage that is greater than the first voltage, the voltage output being coupled to the ion emitter.

The foregoing features and elements may be combined in any combination, without exclusivity, unless expressly indicated herein otherwise, insofar as they fall within the scope of the appended claims. These features and elements as well as the operation of the disclosed embodiments will become more apparent in light of the following description and accompanying drawings.

### BRIEF DESCRIPTION OF THE DRAWINGS

The subject matter of the present disclosure is particularly pointed out and distinctly claimed in the concluding portion of the specification. A more complete understanding of the present disclosure, however, may best be obtained by referring to the following detailed description and claims in connection with the following drawings. While the drawings illustrate various embodiments employing the principles described herein, the drawings do not limit the scope of the claims.
FIG. 1 illustrates an aircraft and various sections within the aircraft, in accordance with various embodiments.
FIGS. 2A and 2B illustrate a galley in an aircraft with an integrated air ionizer, in accordance with various embodiments.
FIGS. 3A and 3B illustrate a galley in an aircraft with an integrated air ionizer, in accordance with various embodiments.
FIGS. 4A, 4B, and 4C illustrate ion emitters installed on an air outlet within a galley with an integrated air ionizer, in accordance with various embodiments.
FIG. 5 illustrates a user interface for controlling an air ionizer in a galley, in accordance with various embodiments.
FIG. 6 illustrates a system for controlling an air ionizer in a galley, in accordance with various embodiments.

### DETAILED DESCRIPTION

The following detailed description of various embodiments herein makes reference to the accompanying drawings, which show various embodiments by way of illustration. While these various embodiments are described in sufficient detail to enable those skilled in the art to practice the disclosure, it should be understood that other embodiments may be realized and that changes may be made without departing from the scope of the invention as defined by the claims. Thus, the detailed description herein is presented for purposes of illustration only and not of limitation. While these exemplary embodiments are described in sufficient detail to enable those skilled in the art to practice the invention, it should be understood that other embodiments may be realized and that logical, chemical and mechanical changes may be made without departing from the scope of the appended claims.

For example, the steps recited in any of the method or process descriptions may be executed in any order and are not necessarily limited to the order presented. Furthermore, any reference to singular includes plural embodiments, and any reference to more than one component or step may include a singular embodiment or step. Also, any reference to attached, fixed, connected, or the like may include permanent, removable, temporary, partial, full or any other possible attachment option. Additionally, any reference to without contact (or similar phrases) may also include reduced contact or minimal contact. It should also be understood that unless specifically stated otherwise, references to "a," "an" or "the" may include one or more than one and that reference to an item in the singular may also include the item in the plural. Further, all ranges may include upper and lower values and all ranges and ratio limits disclosed herein may be combined.

Disclosed herein is galley including an integrated air ionizer. The air ionizer, in various embodiments, includes a control unit and one or more ion emitters. In various embodiments, the ion emitters may be coupled to an air outlet located within the galley. In various embodiments the ion emitters may be arranged perpendicular to a flow of air through the air outlet. In various embodiments, the ion emitters may be located radially around the air outlet. In various embodiments, the ion emitters are positioned to direct ions toward the passenger space proximate the galley. In various embodiments, the integrated air ionizer provides a high density of ions into the passenger area to combat chemicals and compounds that may cause malodor within the galley.

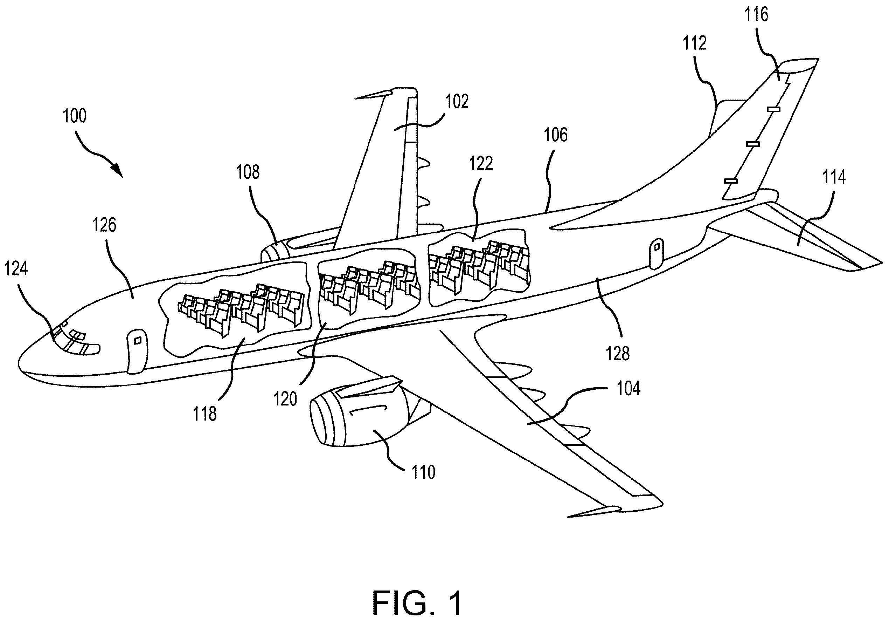

Referring now to FIG. 1, an aircraft 100 and various sections within the aircraft is illustrated, in accordance with various embodiments. Aircraft 100 is an example of a passenger or transport vehicle in which smart air ionizers may be implemented in accordance with various embodiments. In various embodiments, aircraft 100 has a starboard wing 102 and a port wing 104 attached to a fuselage 106. In various embodiments, aircraft 100 also includes a starboard engine 108 connected to starboard wing 102 and a port engine 110 connected to port wing 104. In various embodiments, aircraft 100 also includes a starboard horizontal stabilizer 112, a port horizontal stabilizer 114, and a vertical stabilizer 116. In various embodiments, aircraft 100 also includes various cabin sections, including, for example, a first cabin section 118, a second cabin section 120, a third cabin section 122, and a pilot cabin 124. In various embodiments, aircraft 100 may include a front galley 126 and/or a rear galley 128.

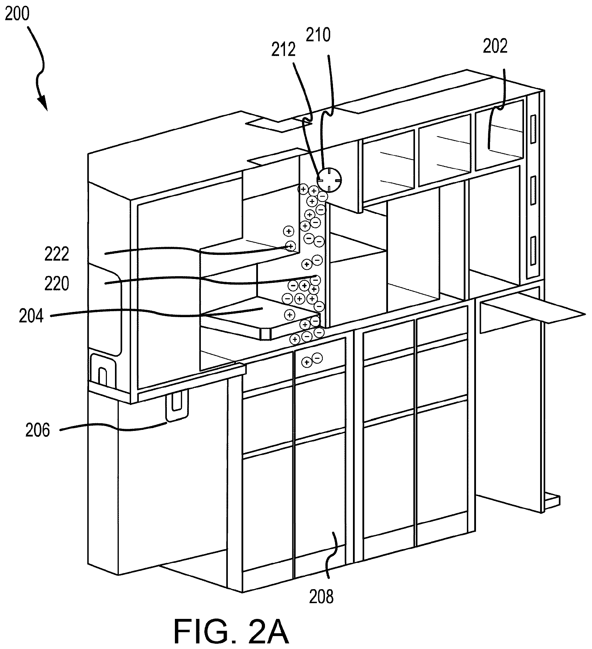

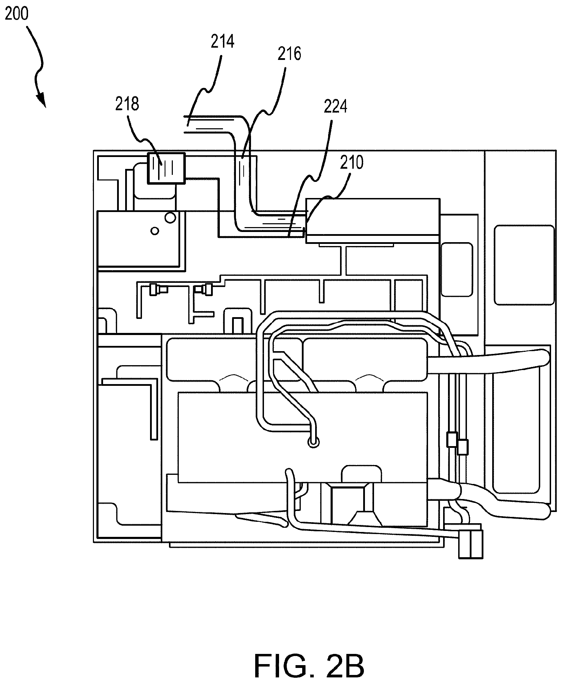

Referring now to FIGS. 2A and 2B, a galley 200 is illustrated, in accordance with various embodiments. In various embodiments, the galley 200 may be an example of the front galley 126 or the rear galley 128 of FIG. 1. FIG. 2A illustrates a front view of the galley 200. FIG. 2B illustrates a rear view of the galley 200. In various embodiments, the galley 200 may include a plurality of stowage bins 202, a preparation area 204, a trash receptacle 206, one or more galley carts 208, an air outlet 210, ion emitters 212, an air inlet 214, an air duct 216 coupled to air inlet 214 and air outlet 210, and a control unit 218. In various embodiments, the ion emitters 212 may be mounted on a side wall proximate the preparation area 204, adjacent the air outlet 210, within the air outlet 210, or at an outlet of the air outlet 210. In various embodiments, the ion emitters 212 may be mounted within the air outlet 210. In various embodiments, the ion emitters 212 generate ionized air particles, including negative ions 220 and positive ions 222, that are spread throughout the galley 200 by air flow from the air outlet 210.

In various embodiments, the ion emitters 212, and more specifically, the positive ions 222 may neutralize pathogens (e.g., bacteria, viruses, molds, dust, etc.) and/or malodor (i.e., unpleasant smells) that are airborne and on surfaces. Pathogens and malodor may be generated and/or spread by the plurality of stowage bins 202, the preparation area 204, the trash receptacle 206, and the one or more galley carts 208, among other locations. Pathogens and malodor may be airborne and/or settle on surfaces within the galley 200, including the plurality of stowage bins 202, the preparation area 204, the trash receptacle 206, and the one or more galley carts 208, among others.

As illustrated in FIGS. 2A and 2B, the ion emitters 212 may be placed adjacent the air outlet 210 so that the ion emitters 212 ionizes the air exiting the air outlet 210. Generally, ions created by the ion emitters 212, such as the negative ions 220 and the positive ions 222, have an active life span of about 30 seconds to about 75 seconds, and more specifically, about 45 seconds to about 60 seconds. Accordingly, locating the ion emitters 212 adjacent the air outlet 210 improves the efficacy of the negative ions 220 and the positive ions 222 as compared to placing an air ionizer within ductwork of an air handling system or adjacent an air filter within the air handling system. In various embodiments, the ion emitters 212 may be placed inside the air outlet 210, and more specifically, within the ductwork behind the air outlet 210 and immediately adjacent the air outlet 210. In various embodiments, the ion emitters 212 may be incorporated into the air outlet 210 so the ion emitters 212 and the air outlet 210 operate as a single unit to ionize and disperse air throughout the galley 200.

In various embodiments, the galley 200 further includes the control unit 218 that is operatively and electrically coupled to the ion emitters 212 by one or more wires 224. In various embodiments, the control unit 218 controls each of the ion emitters 212 by adjusting a voltage, a current, a timing, and/or other parameters to increase or decrease a number of ions produced by each of the ion emitters 212. In various embodiments, increasing a voltage and/or a current to each of the ion emitters 212 increases the number of ions produced by the ion emitters 212. In various embodiments, decreasing the voltage and/or the current to each of the ion emitters 212 decreases the number of ions produced by the ion emitters 212. In various embodiments, the control unit 218 may control each of the ion emitters 212 individually and/or as all together. In various embodiments, the control unit 218 may step up a received alternating current (AC) or a direct current (DC) voltage to a high voltage for use by the ion emitters 212. In various embodiments, the control unit 218 may be connected to a 115 V AC power source. In various embodiments, the control unit 218 may be connected to a 28 V DC power source. In various embodiments, the control unit 218 may output about 2.5 KV to about 12.5 KV, and more specifically, about 5 KV to about 10 KV.

In various embodiments, the control unit 218 may comprise one or more processors configured to implement various logical operations in response to execution of instructions, for example, instructions stored on a non-transitory, tangible, computer-readable medium. The one or more processors can be a general-purpose processor, a microprocessor, a microcontroller, a digital signal processor (DSP), an application specific integrated circuit (ASIC), a field programmable gate array (FPGA) or other programmable logic device, discrete or transistor logic, discrete hardware components, or any combination thereof. In various embodiments, the control unit 218 may further comprise memory to store data, executable instructions, system program instructions, and/or controller instructions to implement the control logic of the control unit 218.

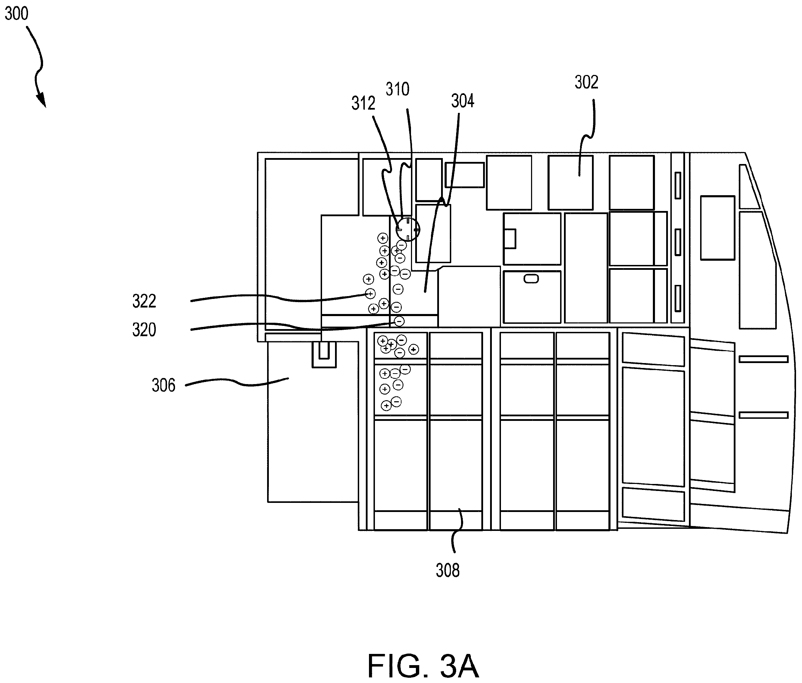

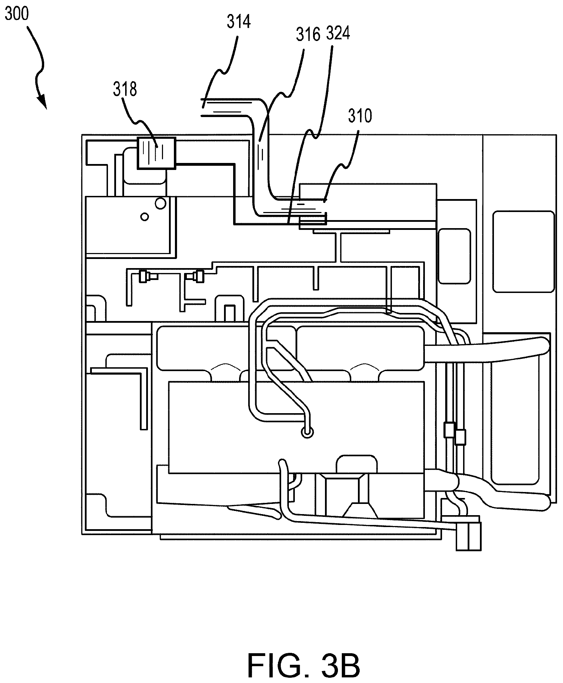

Referring now to FIGS. 3A and 3B, a galley 300 is illustrated, in accordance with various embodiments. In various embodiments, the galley 300 may be an example of the front galley 126 or the rear galley 128 of FIG. 1. FIG. 3A illustrates a front view of the galley 300. FIG. 3B illustrates a rear view of the galley 300. In various embodiments, the galley 300 may include a plurality of stowage bins 302, a preparation area 304, a trash receptacle 306, one or more galley carts 308, an air outlet 310, one or more ion emitters 312, an air inlet 314, an air duct 316 coupled to air inlet 314 and air outlet 310, and a control unit 318. In various embodiments, the ion emitters 312 may be mounted on a back wall proximate the preparation area 304, adjacent the air outlet 310, within the air outlet 310, or at an outlet of the air outlet 310. In various embodiments, the ion emitters 312 may be mounted within the air outlet 310. In various embodiments, the ion emitters 312 generate ionized air particles, including negative ions 320 and positive ions 322, that are spread throughout the galley 300 by air flow from the air outlet 310.

**In** various embodiments, the ion emitters 312, and more specifically, the positive ions 322 may neutralize pathogens (e.g., bacteria, viruses, molds, dust, etc.) and/or malodor (i.e., unpleasant smells) that are airborne and on surfaces. Pathogens and malodor may be generated and/or spread by the plurality of stowage bins 302, the preparation area 304, the trash receptacle 306, and the one or more galley carts 308, among other locations. Pathogens and malodor may be airborne and/or settle on surfaces within the galley 300, including the plurality of stowage bins 302, the preparation area 304, the trash receptacle 306, and the one or more galley carts 308, among others.

As illustrated in FIGS. 3A and 3B, the ion emitters 312 may be placed adjacent the air outlet 310 so that the ion emitters 312 ionizes the air exiting the air outlet 310. Generally, ions created by the ion emitters 312, such as the negative ions 320 and the positive ions 322, have an active life span of about 30 seconds to about 75 seconds, and more specifically, about 45 seconds to about 60 seconds. Accordingly, locating the ion emitters 312 adjacent the air outlet 310 improves the efficacy of the negative ions 320 and the positive ions 322 as compared to placing an air ionizer within ductwork of an air handling system or adjacent an air filter within the air handling system. In various embodiments, the ion emitters 312 may be placed inside the air outlet 310, and more specifically, within the ductwork behind the air outlet 310 and immediately adjacent the air outlet 310. In various embodiments, the ion emitters 312 may be incorporated into the air outlet 310 so the ion emitters 312 and the air outlet 310 operate as a single unit to ionize and disperse air throughout the galley 300.

In various embodiments, the galley 300 further includes the control unit 318 that is operatively and electrically coupled to the ion emitters 312 by one or more wires 324. In various embodiments, the control unit 318 controls each of the ion emitters 312 by adjusting a voltage, a current, a timing, and/or other parameters to increase or decrease a number of ions produced by each of the ion emitters 312. In various embodiments, increasing a voltage and/or a current to each of the ion emitters 312 increases the number of ions produced by the ion emitters 312. In various embodiments, decreasing the voltage and/or the current to each of the ion emitters 312 decreases the number of ions produced by the ion emitters 312. In various embodiments, the control unit 318 may control each of the ion emitters 312 individually and/or as all together. In various embodiments, the control unit 318 may step up a received alternating current (AC) or a direct current (DC) voltage to a high voltage for use by the ion emitters 312. In various embodiments, the control unit 318 may be connected to a 115 V AC power source. In various embodiments, the control unit 318 may be connected to a 28 V DC power source. In various embodiments, the control unit 318 may output about 2.5 KV to about 12.5 KV, and more specifically, about 5 KV to about 10 KV.

In various embodiments, the control unit 318 may comprise one or more processors configured to implement various logical operations in response to execution of instructions, for example, instructions stored on a non-transitory, tangible, computer-readable medium. The one or more processors can be a general-purpose processor, a microprocessor, a microcontroller, a digital signal processor (DSP), an application specific integrated circuit (ASIC), a field programmable gate array (FPGA) or other programmable logic device, discrete or transistor logic, discrete hardware components, or any combination thereof. In various embodiments, the control unit 318 may further comprise memory to store data, executable instructions, system program instructions, and/or controller instructions to implement the control logic of the control unit 318.

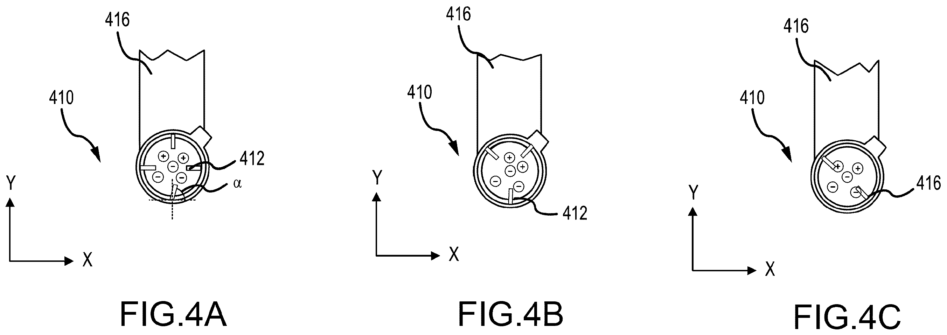

Referring now to FIGS. 4A, 4B, and 4C, ion emitter arrangements around air outlet 210 of FIG. 2A or air outlet 310 of FIG. 3A are illustrated, in accordance with various embodiments. FIG. 4A includes four ion emitters 412, such as ion emitters 212 of FIG. 2A or ion emitters 312 of FIG. 3A disposed radially around an inner diameter of the air outlet 410 of the air duct 416, such as air duct 216 of FIG. 2A or air duct 316 of FIG. 3A. In various embodiments, each of the four of the ion emitters 412 may extend orthogonal to the inner diameter of the air outlet 410 so that each of the ion emitters 412 extends perpendicular to the air flow exiting the air outlet 410. In various embodiments, each of the ion emitters 412 may extend inward from the inner diameter at different non-perpendicular angles, such as angle α illustrated in FIG. 4A. In various embodiments, the ion emitters 412 may be positioned equidistant from each other, as illustrated in FIG. 4A. In various embodiments, the spacing between ion emitters 412 may be non-uniform.

FIG. 4B illustrates three ion emitters 412 disposed radially around an inner diameter of air outlet 410 of air duct 416. In various embodiments, each of the three ion emitters 412 may extend orthogonal to the inner diameter of the air outlet 410 so that each of the ion emitters 412 extends perpendicular to the air flow exiting the air outlet 410. In various embodiments, each ion emitters 412 may extend inward from the inner diameter at different angles. In various embodiments, each of the three ion emitters 412 may be positioned equidistant from each other. In various embodiments, the spacing between one or more of ion emitters 412 may be non-uniform.

FIG. 4C illustrates two of the ion emitters 412 disposed radially around an inner diameter of air outlet 410 of air duct 416. In various embodiments, both ion emitters 412 may extend orthogonal to the inner diameter of air outlet 410 so that each ion emitters 412 extends perpendicular to the air flow exiting the air outlet 410. In various embodiments, each ion emitters 412 may extend inward from the inner diameter at different angles. In various embodiments, both ion emitters 412 may be positioned equidistant from each other. In various embodiments, the spacing between ion emitters 412 may be non-uniform.

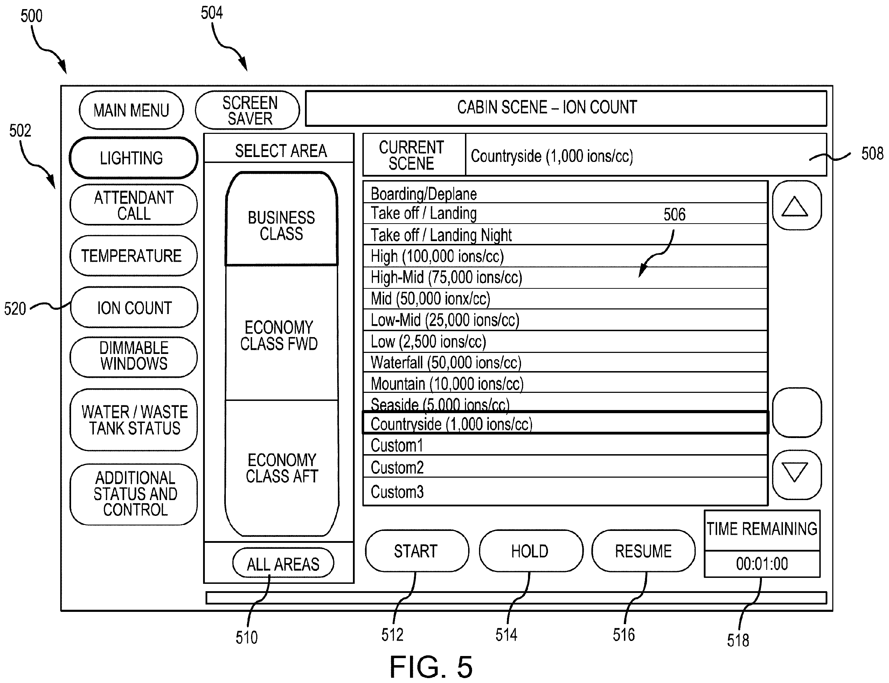

Referring now to FIG. 5, a graphical user interface, GUI 500, for controlling one or more air ionizers is illustrated, in accordance with various embodiments. In various embodiments, GUI 500 may communicate with control unit 218, 318. In various embodiments, the one or more air ionizers may be examples of ion emitters 212, 312, 412 described above in FIGS. 2A, 3A, 4A, 4B, and 4C. It should be appreciated that GUI 500 as described below is not intended to be limiting, but to be exemplary one implementation of a user interface for controlling one or more air ionizers and is for ease of discussion. As such, GUI 500 may be presented differently and/or provide more or less functionality than what is discussed below.

GUI 500 includes an options menu 502, a cabin area selector 505, a scene selector 506, a current scene indicator 508, an all-areas button 510, a start button 512, a hold button 514, a resume button 516, and a timer 518. Options menu 502 provides a crew member the ability to control different aspects of the cabin including lighting, temperature, windows, and ion count and to view status of the cabin including attendant call button and water status. Cabin area selector 504 allow the crew member to select which cabin section to monitor such as business class, economy class forward, and economy class aft. Pressing all areas button 510 may select all cabin areas for adjustment.

Selecting ion count 520 presents the crew member with scene selector 506 and current scene indicator 508. In various embodiments, scene selector 506 may present the crew member with different scenes to adjust the number of ions being generated by air ionizers and ion emitters (e.g., ion emitters 212, 312, 412) within each cabin area. As illustrated, scene selector 506 presents the cabin crew with different options such as "Boarding/Deplane", "Take off / Landing", "Take off / Landing Night", "High", "High-Mid", "Mid", "Mid-Low", "Low", "Waterfall", "Mountain", "Seaside", "Countryside", "Custom1", "Custom2", and "Custom3". The selections in scene selector 506 are intended to simplify the control of the air ionizers for the cabin crew. As illustrated, the different scenes may produce different numbers of ions depending on the current state of the cabin area. For example, the cabin crew may select "Countryside" or "Low" in response to normal conditions within the cabin area. The cabin crew may select "High" or "Waterfall" in response to a malodor within the cabin area (e.g., a beverage maker, garbage, etc.). Doing so provides the cabin crew the ability to adjust the number of ions per cubic centimeter (ions/cc) per unit time to a level that is comfortable for the passengers by increasing or decreasing the voltage supplied to each ion emitter (e.g., ion emitters 212, 312, 412).

Start button 512 may change the selected scene. Hold button 514 may pause the selected scene for a time. Resume button 516 may resume the selected scene after being on hold. Timer 518 may provide an indication of how long the selected scene has been running or on hold. In various embodiments, GUI 500 may be used to control ion output in each galley independently from other gallies. That is, a first scene may be selected for a first galley and a second scene may be selected for a second galley. This allows for individual control of the different gallies based on the use of each galley.

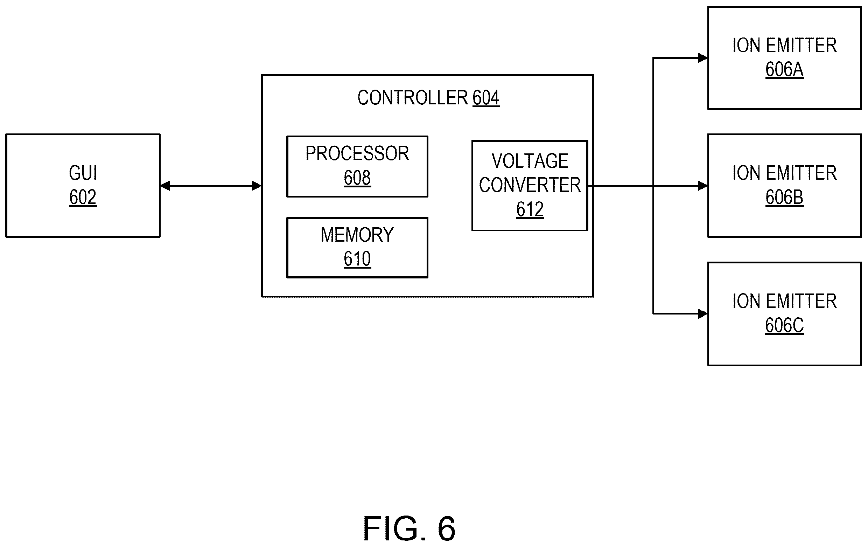

Referring now to FIG. 6, a system 600 for controlling one or more air ionizers is illustrated, in accordance with various embodiments. System 600 includes a graphical user interface (GUI) 602, a controller 604, and ion emitters 606a-606c. In various embodiments, GUI 602 may be an example of GUI 400 described above in FIG. 4. In various embodiments, controller 604 may be an example of control unit 218, 318 described above in FIGS. 2B and 3B. In various embodiments, controller 604 may be an example of a central aircraft controller or other server. In various embodiments, ion emitters 606a-606c may be examples of ion emitters 212, 312, 412 described above in FIGS. 2A, 3A, 4A, 4B, and 4C.

Controller 604 includes a processor 608, a memory 610, and a voltage converter 612. Processor 608 is configured to communicate with GUI 602 to receive commands from a crew member and to control the ion emitters 606a-606c in response to the received commands. Memory 610 is configured to store data from GUI 602 and processor 608. Voltage converter 612 is configured to receive commands from processor 608 and convert an input voltage (e.g., 28 VDC, 115 VAC) to a high voltage output (e.g., 10 kV DC) for use by ion emitters 606a-606c).

Processor 608 may comprise one or more processors configured to implement various logical operations in response to execution of instructions, for example, instructions stored on a non-transitory, tangible, computer-readable medium. The one or more processors can be a general-purpose processor, a microprocessor, a microcontroller, a digital signal processor (DSP), an application specific integrated circuit (ASIC), a field programmable gate array (FPGA) or other programmable logic device, discrete or transistor logic, discrete hardware components, or any combination thereof. Memory 610 may comprise memory to store data, executable instructions, system program instructions, and/or controller instructions to implement the control logic of processor 608.

System program instructions and/or controller instructions may be loaded onto a non-transitory, tangible computer-readable medium having instructions stored thereon that, in response to execution by a controller, cause the controller to perform various operations. The term "non-transitory" is to be understood to remove only propagating transitory signals per se from the claim scope and does not relinquish rights to all standard computer-readable media that are not only propagating transitory signals per se.

Benefits, other advantages, and solutions to problems have been described herein with regard to specific embodiments. Furthermore, the connecting lines shown in the various figures contained herein are intended to represent exemplary functional relationships and/or physical couplings between the various elements. It should be noted that many alternative or additional functional relationships or physical connections may be present in a practical system. However, the benefits, advantages, solutions to problems, and any elements that may cause any benefit, advantage, or solution to occur or become more pronounced are not to be construed as critical, required, or essential features or elements of the disclosure. The scope of the disclosure is accordingly to be limited by nothing other than the appended claims, in which reference to an element in the singular is not intended to mean "one and only one" unless explicitly so stated, but rather "one or more." Moreover, where a phrase similar to "at least one of A, B, or C" is used in the claims, it is intended that the phrase be interpreted to mean that A alone may be present in an embodiment, B alone may be present in an embodiment, C alone may be present in an embodiment, or that any combination of the elements A, B and C may be present in a single embodiment; for example, A and B, A and C, B and C, or A and B and C. Different cross-hatching is used throughout the figures to denote different parts but not necessarily to denote the same or different materials.

Systems, methods, and apparatus are provided herein. In the detailed description herein, references to "one embodiment," "an embodiment," "various embodiments," etc., indicate that the embodiment described may include a particular feature, structure, or characteristic, but every embodiment may not necessarily include the particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to affect such feature, structure, or characteristic in connection with other embodiments whether or not explicitly described. After reading the description, it will be apparent to one skilled in the relevant art(s) how to implement the disclosure in alternative embodiments.

Numbers, percentages, or other values stated herein are intended to include that value, and also other values that are about or approximately equal to the stated value, as would be appreciated by one of ordinary skill in the art encompassed by various embodiments of the present disclosure. A stated value should therefore be interpreted broadly enough to encompass values that are at least close enough to the stated value to perform a desired function or achieve a desired result. The stated values include at least the variation to be expected in a suitable industrial process, and may include values that are within 5% of a stated value. Additionally, the terms "substantially," "about" or "approximately" as used herein represent an amount close to the stated amount that still performs a desired function or achieves a desired result. For example, the term "substantially," "about" or "approximately" may refer to an amount that is within 5% of a stated amount or value.

Finally, it should be understood that any of the above-described concepts can be used alone or in combination with any or all of the other above-described concepts. Although various embodiments have been disclosed and described, one of ordinary skill in this art would recognize that certain modifications would come within the scope of this invention as defined by the claims. Accordingly, the description is not intended to be exhaustive or to limit the principles described or illustrated herein to any precise form. Many modifications and variations are possible in light of the above teaching, insofar as they fall within the scope of the appended claims.

## Claims

1. An aircraft galley, comprising:
an air duct (216) including an air inlet (214) and an air outlet (210), the air outlet extending into the aircraft galley, the air outlet (210) having an opening having an outer circumference;
an ion emitter (212); and
a control unit (218) electronically coupled to the ion emitter, the control unit configured to control an ion output of the ion emitter;
**characterised in that** the ion emitter is coupled to the outer circumference of the opening and extends radially inward from the outer circumference.

2. The aircraft galley of claim 1, wherein the ion emitter (212) is configured to be in an air flow passing through the air outlet.

3. The aircraft galley of claim 2, wherein the ion emitter is perpendicular to the air flow passing through the air outlet.

4. The aircraft galley of claim 1, further comprising:
a plurality of ion emitters arranged around the outer circumference of the opening, wherein each of the plurality of ion emitters is perpendicular to a flow of air passing through the opening.

5. The aircraft galley of any preceding claim, wherein the air inlet extends to an exterior of the aircraft galley to provide the air duct with air from outside of the aircraft galley.

6. The aircraft galley of any preceding claim, wherein the control unit comprises:
a voltage input having a first voltage; and
a voltage output having a second voltage that is greater than the first voltage, the voltage output being coupled to the ion emitter.

7. An aircraft, comprising:
a galley (126, 128) as claimed in any preceding claim.

## Patentansprüche

1. Luftfahrzeugküche, umfassend:
einen Luftkanal (216), beinhaltend einen Lufteinlass (214) und einen Luftauslass (210), wobei sich der Luftauslass in die Luftfahrzeugküche erstreckt, wobei der Luftauslass (210) eine Öffnung aufweist, die einen Außenumfang aufweist;
einen Ionenemitter (212); und
eine Steuereinheit (218), die elektronisch mit dem Ionenemitter gekoppelt ist, wobei die Steuereinheit konfiguriert ist, einen Ionenausstoß des Ionenemitters zu steuern;
**dadurch gekennzeichnet, dass**
der Ionenemitter mit dem Außenumfang der Öffnung gekoppelt ist und sich radial nach innen von dem Außenumfang erstreckt.

2. Luftfahrzeugküche nach Anspruch 1, wobei der Ionenemitter (212) konfiguriert ist, sich in einem Luftstrom zu befinden, der durch den Luftauslass hindurchgeht.

3. Luftfahrzeugküche nach Anspruch 2, wobei der Ionenemitter senkrecht zu dem Luftstrom ist, der durch den Luftauslass hindurchgeht.

4. Luftfahrzeugküche nach Anspruch 1, ferner umfassend:
eine Mehrzahl von Ionenemittern, die um den Außenumfang der Öffnung herum angeordnet sind, wobei jeder der Mehrzahl von Ionenemittern senkrecht zu einem Luftstrom ist, der durch die Öffnung hindurchgeht.

5. Luftfahrzeugküche nach einem der vorhergehenden Ansprüche, wobei sich der Lufteinlass zu einer Außenseite der Luftfahrzeugküche erstreckt, um den Luftkanal mit Luft von außerhalb der Luftfahrzeugküche zu versorgen.

6. Luftfahrzeugküche nach einem der vorhergehenden Ansprüche, wobei die Steuereinheit umfasst:
einen Spannungseingang, der eine erste Spannung aufweist; und
einen Spannungsausgang, der eine zweite Spannung aufweist, die größer als die erste Spannung ist, wobei der Spannungsausgang mit dem Ionenemitter gekoppelt ist.

7. Luftfahrzeug, umfassend:
eine Küche (126, 128) nach einem der vorhergehenden Ansprüche.

## Revendications

1. Cuisine de bord d'aéronef, comprenant :
un conduit d'air (216) incluant une entrée d'air (214) et une sortie d'air (210), la sortie d'air s'étendant dans la cuisine d'aéronef, la sortie d'air (210) présentant une ouverture présentant une circonférence extérieure ;
un émetteur d'ions (212) ; et
une unité de commande (218) couplée électroniquement à l'émetteur d'ions, l'unité de commande étant configurée pour contrôler une sortie d'ions de l'émetteur d'ions ;
**caractérisée en ce que**
l'émetteur d'ions est couplé à la circonférence extérieure de l'ouverture et s'étend radialement vers l'intérieur à partir de la circonférence extérieure.

2. Cuisine de bord d'aéronef selon la revendication 1, dans laquelle l'émetteur d'ions (212) est configuré pour être dans un flux d'air passant à travers la sortie d'air.

3. Cuisine de bord d'aéronef selon la revendication 2, dans laquelle l'émetteur d'ions est perpendiculaire au flux d'air passant à travers la sortie d'air.

4. Cuisine de bord d'aéronef selon la revendication 1, comprenant en outre :
une pluralité d'émetteurs d'ions disposés autour de la circonférence extérieure de l'ouverture, chacun de la pluralité des émetteurs d'ions étant perpendiculaire à un flux d'air passant à travers l'ouverture.

5. Cuisine de bord d'aéronef selon l'une quelconque des revendications précédentes, dans laquelle l'entrée d'air s'étend à l'extérieur de la cuisine d'aéronef pour fournir au conduit d'air de l'air provenant de l'extérieur de la cuisine d'aéronef.

6. Cuisine de bord d'aéronef selon l'une quelconque des revendications précédentes, dans laquelle l'unité de commande comprend :
une entrée de tension présentant une première tension ; et
une sortie de tension présentant une seconde tension qui est supérieure à la première tension, la sortie de tension étant couplée à l'émetteur d'ions.

7. Aéronef, comprenant :
une cuisine de bord (126, 128) selon l'une quelconque des revendications précédentes.
